# FASCICULE DE BREVET EUROPEEN

(11) **EP 0 469 102 B1**
(45) Date de publication et mention de la délivrance du brevet: **01.03.1995**
(21) Numéro de dépôt: 91902722.7
(22) Date de dépôt: 31.12.1990
(51) Int. Cl.: C07K 5/08, G01N 33/68, C12Q 1/56

(54) **NOUVEAUX SUBSTRATS PEPTIDIQUES, PROCEDE DE PREPARATION ET UTILISATION DANS LA DETERMINATION DE LA PROTEINE C**
NEUE PEPTIDVERBINDUNGEN, VERFAHREN ZUR HERSTELLUNG UND IHRE VERWENDUNG ZUM NACHWEIS VON PROTEIN C
NOVEL PEPTIDE SUBSTRATES, METHOD OF PREPARATION AND USE IN C PROTEIN DETERMINATION

(30) Priorité: 19.02.1990 FR 9001966
(43) Date de publication de la demande: 05.02.1992
(73) Titulaire: SERBIO, F-92230 Gennevilliers (FR)
(72) Inventeur: QUENTIN, Gérard, F-92700 Colombes (FR); MARTINOLI, Jean-Luc, F-92390 Villeneuve-la-Garenne (FR)
(74) Mandataire: Clisci, Serge
(86) Numéro de dépôt international: FR9000973
(87) Numéro de publication internationale: WO9112268

(56) Documents cités:
- EP-A- 0 004 256
- EP-A- 0 110 306
- EP-A- 0 280 610
- US-A- 4 335 210

## Description

### DOMAINE DE L'INVENTION

La présente invention concerne en tant que produits industriels nouveaux les substrats peptidiques de formule I ci-après et leurs sels d'addition.

Elle concerne également le procédé de préparation de ces nouveaux produits et leur utilisation dans le domaine de la détermination de la Protéine C.

### ART ANTERIEUR

On sait que l'on a déjà proposé dans le passé un certain nombre de substrats peptidiques pour l'identification et le dosage de nombreuses substances intervenant dans le mécanisme en cascade de l'hémostase.

On sait en particulier que le substrat
H-L-Pyr-L-Pro-L-Arg-pNA (IIa)
[où Pyr représente le reste pyroglutaminyle (i.e. 5-oxo-prolyle ou 2-pyrrolidinone-5-carbonyle) et pNA le reste p-nitroanilino], décrit dans EP-A-0 004 256 en tant que substrat chromogène pour la détermination de sérine protéases et de SH-protéases, s'est révélé récemment efficace dans la détermination de la Protéine C.

On sait également que le substrat
H-D-Lys(Cbo)-L-Pro-L-Arg-pNA (IIb)
a déjà été utilisé dans le domaine de la détermination de la Protéine C mais que son efficacité est inférieure à celle du composé IIa précité.

### BUT DE L'INVENTION

Il existe donc un besoin en substrats peptidiques spécifiques pour la détermination de la Protéine C. En effet, à l'exclusion des substrats IIa et IIb précités, il n'existe aucun autre substrat spécifique de la Protéine C qui soit commercialement disponible.

Pour satisfaire ce besoin, on propose suivant l'invention une nouvelle solution technique faisant appel à des substrats peptidiques structurellement différents des produits de formule IIa et IIb précités antérieurement connus.

### OBJET DE L'INVENTION

Selon un premier aspect de l'invention, on préconise en tant que produits industriels nouveaux des substrats peptidiques caractérisés en ce qu'ils sont choisis parmi l'ensemble constitué par
(i) les tripeptides de formule

   Y-A₁-A₂-A₃-R (I)

   dans laquelle
   Y représente H ou un groupe approprié bloquant l'extrémité N-terminale,
   A₁ représente un reste aminoacide choisi parmi l'ensemble constitué par les restes (2-oxo-thiazolidin-4-yl)carbonyle [en abré gé : THC], (2-oxo-tétrahydro-1,3-thiazin-5-yl)carbonyle [en abrégé : TZC], thiazolidine-4-carbonyle [en abrégé : ATC] et (tétrahydro-1,3-thiazin-5-yl)carbonyle [en abrégé = AZC],
   A₂ représente un reste aminoacide choisi parmi l'ensemble constitué par les restes Pro, 3Hyp et 4Hyp, où le groupe OH latéral de 3Hyp et 4Hyp est susceptible d'être protégé par un groupe protecteur éther ou ester,
   A₃ représente un reste aminoacide choisi parmi l'ensemble constitué par les restes Arg et Lys, et R représente un moyen de marquage; et
(ii) leurs sels d'addition.

Selon un second aspect de l'invention, on préconise l'utilisation des composés tripeptides de formule I et de leurs sels d'addition pour la détermination de la Protéine C. Dans cette optique, on recommande un procédé de détermination pour le dosage ou l'identification de la Protéine C comprenant la mise en contact d'un tripeptide de formule I ou l'un de ses sels d'addition avec un liquide corporel (notamment le plasma ou le sérum sanguin, ou encore l'urine) ou tout autre échantillon d'origine naturelle ou synthétique susceptible de contenir de la Protéine C.

Selon un troisième aspect de l'invention, on préconise enfin un procédé de préparation des composés tripeptidiques de formule I et de leurs sels d'addition.

### ABREVIATIONS

Dans la présente invention, on a utilisé par commodité les abréviations suivantes :
- pour les restes aminoacides
   - Arg: = arginyle
   - ATC: = thiazolidine-4-carbonyle (ou thioprolyle)
   - AZC: = (tétrahydro-1,3-thiazin-5-yl)carbonyle
   - 3Hyp: = 3-hydroxyprolyle (ou 3-hydroxy-2-pyrrolidinecarbonyle
   - 4Hyp: = 4-hydroxyprolyle (ou 4-hydroxy-2-pyrrolidinecarbonyle
   - Lys: = lysyle
   - Pro: = prolyle
   - Pyr: = pyroglutaminyle (ou 2-pyrrolidone-5-carbonyle)
   - THC: = (2-oxo-thiazolidin-4-yl)carbonyle
   - TZC: =(2-oxo-tétrahydro-1,3-thiazin-5-yl)carbonyle
- pour les autres abréviations
   - Ac: = acétyle
   - AcOH: = acide acétique
   - Adoc: = adamantyloxycarbonyle
   - Aoc: = t-amyloxycarbonyle
   - Boc: = t-butyloxycarbonyle
   - Bop: = hexafluorophosphate de 1-(benzotriazolyl)oxy-tris(diméthylamino)phosphonium (autre nomenclature réactif de CASTRO) de formule 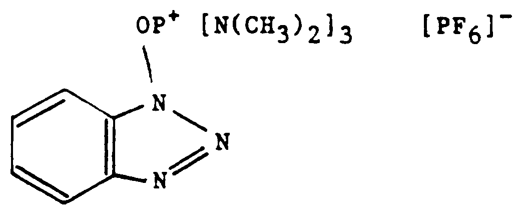
   - Bu: = n-butyle
   - Bz: = benzoyle
   - Bzl: = benzyle
   - Cbo: = carbobenzoxy
   - CCM: = chromatographie sur couche mince
   - o-Cl-pNA: = o-chloro-p-nitroanilino [ou (2-Cl)pNA]
   - DCCI: = dicyclohexylcarbodiimide
   - DIEA: = diisopropyléthylamine
   - DMF: = diméthylformamide
   - Et: = éthyle
   - Et₃N: = triéthylamine
   - EtO: = éthoxy
   - Fmoc: = 9-fluroenylméthyloxycarbonyle
   - Foc: = furfuryloxycarbonyle
   - HOBT: = 1-hydroxybenzotriazole
   - HMPT: = N,N,N',N',N'',N''-hexaméthylphosphorotriamide
   - HPLC: = chromatographie liquide haute performance
   - H-TFA: = acide trifluoroacétique (ou encore HTFA)
   - Iboc: = isobornyloxycarbonyle
   - iPr: = isopropyle
   - Me: = méthyle
   - OD: = densité optique
   - Ph: = phényle
   - pH: = cologarithme de la concentration en ions H⁺
   - PM: = poids moléculaire
   - pNA: = p-nitroanilino [ou (4-NO₂)C₆H₄NH]
   - Pr: = n-propyle
   - RT: = température ambiante (15-20°C)
   - tBu: = t-butyle
   - THF: = tétrahydrofuranne
   - Tos: = p-toluènesulfonyle (ou tosyle)
   - Z: = benzyloxycarbonyle
   - Z(p-Cl): = p-chlorobenzyloxycarbonyle
   - Z(p-OMe): = p-méthoxybenzyloxycarbonyle.

### DESCRIPTION DETAILLEE DE L'INVENTION

Dans la formule I ci-dessus, le reste aminoacide A₁ peut être représenté par la formule développée suivante 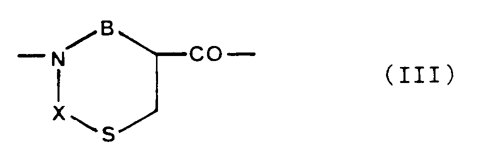
dans laquelle
- B: représente une simple liaison ou le groupe CH₂, et
- X: représente CH₂ ou CO.

Lorsque dans la formule III, le couple (B;X) représente (-; CO), (CH₂;CO), (-;CH₂) et respectivement (CH₂;CH₂), A₁ représente THC, TZC, ATC et respectivement AZC.

Le reste aminoacide A₁ sera suivant l'invention de configuration D ou L ou encore un mélange racémique (DL) de ces deux configurations. De préférence, A₁ représentera D-THC, D-TZC, D-ATC, D-AZC et mieux L-THC, L-TZC, L-ATC et L-AZC. Le reste aminoacide A₁ que l'on considère comme le plus intéressant suivant l'invention est L-THC.

Parmi les groupes Y qui conviennent, on peut mentionner l'atome d'hydrogène et les restes bloquants l'extrémité N-terminale de peptides tels que décrits dans EP-A-0 110 306, US-A-4 480 030, FR-A-2 293 439 et US-A-4 440 678. Parmi les groupes Y, qui sont différents de H, bloquant ladite extrémité N-terminale on peut notamment signaler les groupes alkyle en C₁-C₄ (notamment Me, Et, Pr, iPr, Bu, tBu), aryle éventuellement substitué (notamment Ph, tolyle, xylyle, chlorophényle, trifluorométhylphényle, méthoxyphényle), aralkyle éventuellent substitué (notamment Bzl, chlorobenzyle, dichlorobenzyle, trifluorométhylbenzyle, difluorobenzyle, méthoxybenzyle, éthoxybenzyle, 3,4-méthylènedioxybenzyle) et les groupes protecteurs classiques de l'extrémité N-terminale de peptides [notamment Ac, Tos, Adoc, Aoc, Bz, Cbo, Fmoc, Foc, Iboc, Z, Z(p-Cl) et Z(p-OMe)].

Le groupe Y différent de H est introduit dans le tripeptide de formule I selon l'un des deux schémas réactionnels suivants : 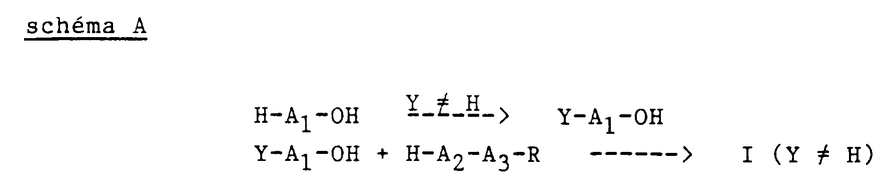
où le groupe OH (de la fonction acide carboxylique) lié à l'extrémité CO-terminale de A₁ est le cas échéant protégé; et 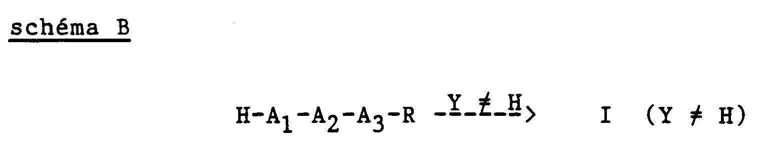
Le choix du schéma A ou B sera fonction de (i) la nature du groupe bloquant, et (ii) du caractère labile de l'atome d'hydrogène de l'extrémité N-terminale qui se trouve en position 3 du cycle de A₁. Dans cette optique, quand A₁ sera THC ou TZC, on utilisera plutôt le schéma A.

Par ailleurs, comme les cycles de THC et TZC sont en général obtenus par cyclisation à température élevée, il se trouve que dans les mécanismes réactionnels par pyrolyse : 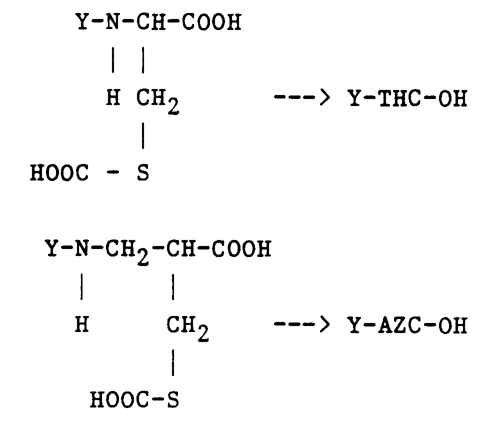
la présence d'un groupe Y différent de H, notamment H = alkyle, aryle ou aralkyle, peut perturber la cyclisation et/ou diminuer les rendements. On a donc intérêt à préparer les acides H-THC-OH et H-AZC-OH par pyrolyse, puis à remplacer l'atome H de l'extrémité N-terminale selon le schéma A précité pour obtenir les composés de formule I où Y est différent de H.

Le groupe Y préféré suivant l'invention est H. En bref, on remplacera le groupe Y=H par un groupe Y différent de H que si et seulement si un tel remplacement augmente de façon substantielle la spécificité du substrat Y-A₁-A₂-A₃-R vis-à-vis de la Protéine C.

Comme indiqué plus haut, le groupe OH latéral de 3Hyp et 4Hyp peut être protégé sous forme éther ou ester. Le groupe protecteur remplaçant l'atome H dudit groupe OH pourra notamment être un groupe alkyle en C₁-C₄ (notamment Me, Et, Pr, iPr, Bu, tBu), aryle éventuellement substitué (notamment Ph, tolyle, xylyle, chlorophényle, trifluorométhylphényle, méthoxyphényle), aralkyle éventuellement substitué (notamment Bzl, chlorobenzyle, difluorobenzyle, trifluorométhylbenzyle, dichlorobenzyle, méthoxybenzyle, éthoxybenzyle, 3,4-méthylènedioxybenzyle) tel qu'envisagé dans la défintion de Y, dans le cadre de la protection sous forme éther; ou par un groupe acyle aliphatique en C₂-C₅ (notamment Ac, propionyle, butanoyle, pentanoyle) ou aromatique (notamment Bz, toluoyle, chlorobenzoyle, méthoxybenzoyle, 3,4-méthylènedioxybenzoyle), dans le cadre de la protection sous forme ester. Le cas échéant, ledit atome H pourra également être remplacé par un groupe Tos ou trialkylsilyle (notamment triméthylsilyle ou triéthylsilyle) ou analogue.

De façon avantageuse, les restes aminoacides A₂ et A₃ seront de configuration L, et les groupes préférés pour A₂ et respectivement A₃ suivant l'invention seront L-Pro, L-3Hyp et L-4Hyp, et respectivement L-Arg et L-Lys.

Le moyen de marquage R est bien connu dans la technique des dosages biologiques et microbiologiques, voir à cet effet l'art antérieur précité et notamment le document US-A-4 448 715. Ledit moyen de marquage sera de préférence choisi parmi l'ensemble des groupes aminés NH-R' qui (i) induisent une modification de coloration, (ii) induisent une modification de fluorescence ou (iii) comportent au moins un élément radioactif (par exemple un groupe anilino ou benzylamino marqué par un radiosotope ¹⁴C ou ³H). Convient au but de l'invention tout groupe amino NH-R' donnant pendant ou après la réaction enzymatique un signal susceptible d'être amplifié pour être détecté (par exemple par mesure de la densité optique sous une longueur d'onde donnée, ou encore par mesure de la radioactivité). La quantité de produit H-R obtenu par clivage lors de l'hydrolyse enzymatique est proportionnelle à la quantité d'enzyme utilisée. Ladite quantité de H-R est déterminable photométriquement, spectrophotométriquement, fluorospectrophotométriquement ou électrochimiquement.

Le groupe R que l'on préfère suivant l'invention est un groupe chromogène, de façon typique un groupe nitrophényle (dans lequel le reste phényle est susceptible d'être substitué par un groupe COOH, F, Cl, Br, CH₃, OCH₃, CN, CF₃, et/ou SO₃H), ou un groupe fluorogène, de façon typique un groupe naphtyle (dans lequel le reste naphtyle est susceptible d'être substitué par un groupe OCH₃, COOH, SO₃H ou CH₃), et les groupes 4-méthylcoumaryl-7-amino, 4-trifluorométhylcoumaryl-7-amino et analogues.

Parmi les groupes aminés chromogènes et fluorogènes, qui convienent suivant l'invention, on peut notamment citer les p-nitroanilino (en abrégé pNA), 2-carboxy-4-nitroanilino et 3-carboxy-4-nitroanilino, 2-halogéno-4-nitroanilino et 3-halogéno-4-nitroanilino (où l'halogène est F, Cl ou Br), 2-méthoxy-5-méthyl-4-nitroanilino, 2-hydroxysulfonyl-4-nitroanilino, 4-trifluorométhyl-2-nitroanilino, 4-trifluorométhyl-3-nitroanilino, 4-cyano-2-nitroanilino, 2-naphtylamino, 4-hydroxysulfonyl-1-naphtylamino, quinolylamino, nitroquinolylamino et analogues.

Le groupe R préféré suivant l'invention est un groupe chromogène à savoir, d'une part, pNA et d'autre part, les groupes analogues, où le noyau phényle de pNA est substitué en position 2 ou 3, et répondant à la formule 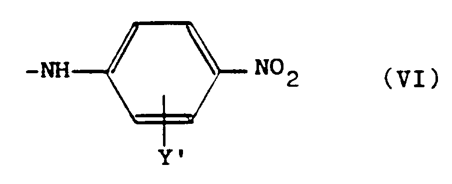
dans laquelle Y' est Br, Cl, F, CF₃, COOH, COOW, CONH₂, CONHW, CONW₂, CONH(CH₂)ₘNMe₂, OH ou OW, où W est un groupe alkyle en C₃-C₆, aryle en C₆-C₁₀, aralkyle en C₇-C₁₁ ou alicyclique en C₃-C₈ et m est un nombre entier ayant pour valeur 1 à 10.

De tels groupes de formule VI où le noyau phényle du groupe pNA est substitué sont notamment décrits dans le document EP-A-0 110 306.

Les sels d'addition suivant l'invention sont essentiellement des sels d'addition d'acide obtenus par réaction d'un composé de formule I avec un acide minéral ou organique.

Le meilleur mode de mise en oeuvre de l'invention, que l'on recommande ici, consiste à faire appel à un substrat choisi parmi l'ensemble constitué par
(a) les composés tripeptidiques de formule

   H-A₁-A₂-A₃-pNA (I')

   dans laquelle
   A₁ représente D-THC, D-TZC, D-ATC, D-AZC, L-THC, L-TZC, L-ATC ou L-AZC,
   A₂ représente L-Pro, L-3Hyp ou L-4Hyp, et
   A₃ représente L-Arg ou L-Lys; et
(b) leurs sels d'addition.

Suivant ce meilleur mode de mise en oeuvre, on recommande plus particulièrement de faire appel à un reste aminoacide A₁ représentant L-TZC, L-ATC et mieux L-THC.

Par commodité, dans la description qui va suivre, la mention d'un reste aminoacide sans configuration (par exemple Pro), désigne sauf indication contraire que ledit reste aminoacide est de configuration L (soit, dans l'exemple en question L-Pro).

De façon nullement limitative, on a consigné dans le tableau I ci-après un certain nombre de composés peptidiques suivant l'invention.

**TABLEAU I**

| H-A₁-A₂-A₃-pNA, HX | | | | |
|---|---|---|---|---|
| Composé | A₁ | A₂ | A₃ | HX |
| Ex 1 | THC | Pro | Arg | H-TFA |
| Ex 2 | THC | Pro | Arg | AcOH |
| Ex 3 | THC | 3Hyp | Arg | AcOH |
| Ex 4 | THC | 4Hyp | Arg | AcOH |
| Ex 5 | THC | Pro | Lys | AcOH |
| Ex 6 | TZC | Pro | Arg | AcOH |
| Ex 7 | ATC | Pro | Arg | 2 AcOH |
| Ex 8 | AZC | Pro | Arg | 2 AcOH |
| CP 1 (a) | Pyr | Pro | Arg | AcOH |
| CP 2 (e) | D-Lys(Cbo) | Pro | Arg | 2 AcOH |

| | | | | |
|---|---|---|---|---|
| Notes (a) produit de référence de formule IIa décrit dans EP-A-0 004 256 (b) produit de référence de formule IIb. | | | | |

Les composés tripeptides de formule I et leurs sels d'addition suivant l'invention peuvent être préparés selon une méthode connue en soi par application de mécanismes réactionnels classiques de la synthèse peptidique. Le procédé de préparation que l'on préconise ici est caractérisé en ce qu'il comprend la réaction de 1 mole de dipeptide de formule

H-A₂-A₃-R (IV)

où A₂, A₃ et R sont définis comme indiqué ci-dessus, avec 1,0 à 1,3 moles d'aminoacide de formule

Y-A₃-T (V)

où Y et A₃ sont définis comme indiqué ci-dessus et T représente OH, F, Cl ou Br.

De façon avantageuse, la réaction IV + V = I est effectuée dans un solvant inerte, notamment DMF ou THF, à une température comprise entre 0 et 50°C, notamment à RT. De façon toujours avantageuse, le milieu réactionnel comprendra une base en excès intervenant comme co-solvant et comme accepteur de protons, telle que Et₃N ou DIEA. Lors de la mise en oeuvre de ladite réaction, on incorporera également dans ledit milieu réactionnel un agent couplant, notamment Bop ou HOBT, en particulier lorsque T est OH. Quand on utilisera HOBT comme agent couplant, on aura intérêt à associer au dit agent une quantité appropriée de DCCI.

De façon pratique, on utilisera des rapports molaires IV/Et₃N ou IV/DIEA de 1/2 à 1/4, et quand T = OH des rapports molaires IV/HOBT ou IV/Bop de 1/1 à 1/5; quand on utilisera DCCI, le rapport molaire IV/DCCI sera de l'ordre de 1/1,5-1/2. Les moyens de couplage HOBT et Bop ont pour objet d'activer la fonction acide carboxylique COOH de l'extrémité CO terminale (quand T = OH) et offrent l'avantage de ne pas être racémisants. Le moyen Bop sera utilisé à un pH de 7-8 et le moyen HOBT à un pH de 5 à 8. Eu égard à ses propriétés de couplage, on préférera le Bop au HOBT. Le tripeptide de formule I est isolé du milieu réactionnel de la réaction IV + V = I selon les étapes suivantes :
- évaporation du solvant inerte sous vide jusqu'à siccité,
- précipitation par l'éther (MeOMe ou EtOEt) du résidu d'évaporation ainsi obtenu, puis récupération du précipité par filtration,
- reprise du précipité ainsi filtré dans une quantité minimale d'un mélange CHCl₃ / MeOH /AcOH,
- chromatographie du mélange résultant au moyen dudit solvant CHCl₃/MeOH/AcOH
- combinaison des fractions homogènes résultantes de ladite chromatographie et évaporation dudit solvant.

Les composés tripeptides de formule I et leurs sels d'addition constituent des substrats spécifiques de la Protéine C. Le procédé de détermination de la Protéine C que l'on préconise est caractérisé en ce que l'on met en contact dans un milieu liquide aqueux approprié tel qu'un sérum physiologique tamponné, une quantité donnée d'un tripeptide de formule I ou l'un de ses sels d'addition (à une concentration de l'ordre de 10 mg/ml) et un échantillon à tester (éventuellement dilué) susceptible de contenir de la Protéine C, pendant au moins 0,25 h à une température compris entre RT et 40°C, notamment 37°C.

L'activité des tripeptides suivant l'invention vis-à-vis de la Protéine C a été déterminée selon une des méthodes classiques, telle que décrite dans la publication EP-A-0 280 610 (voir page 14), en appréciant la vitesse d'hydrolyse par la variation de la densité optique dans le temps (ΔOD/min). Les résultats qui ont été obtenus avec des doses équimolaires ont été consignés dans le tableau II ci-après où par commodité, l'activité du produit de référence de formule IIa est précisée comme étant égale à 100 % .

**TABLEAU II**

| **ACTIVITE VIS-A-VIS DE LA PROTEINE C** | |
|---|---|
| Produit | Activité |
| Ex 1 | 148 % |
| Ex 2 | 153 % |
| Ex 3 | 118 % |
| Ex 4 | 111 % |
| Ex 5 | 122 % |
| Ex 6 | 105 % |
| Ex 7 | 110 % |
| Ex 8 | 103 % |
| CP 1 | 100 % |
| CP 2 | 34 % |

Les résultats comparatifs du tableau II mettent en évidence que les tripeptides suivant l'invention sont au moins aussi actifs que le produit de référence CP 1.

On préconise également suivant l'invention un nécessaire, kit ou trousse de dosage destiné à la détermination de la Protéine C renfermant un tripeptide de formule I ou l'un de ses sels d'addition, et le cas échéant, un échantillon étalon de Protéine C (d'origine humaine ou bovine) et de milieux tamponnés de dilution.

D'autres avantages et caractéristiques de l'invention seront mieux compris à la lecture qui va suivre d'exemples de préparation. Ces éléments nullement limitatifs sont donnés à titre d'illustration.

### PREPARATION I

### Obtention de L-THC-L-Pro-L-Arg-pNA, H-TFA (Exemple 1)

### a) Z-L-Arg-pNA, HCl

On dissout 3,44 g (0,01 mole) de Z-L-Arg-OH, HCl dans 20 ml de HMPT anhydre à RT, puis on ajoute 1,39 ml (0,01 mole) de Et₃N à RT sous agitation. A la solution résultante, on ajoute 2,46 g (0,015 mole) de p-nitrophénylisocyanate. Le milieu réactionnel résultant est maintenu sous agitation pendant 24 h à RT, puis est évaporé sous vide et repris avec une quantité minimale de AcOH avant d'être dilué avec AcOEt. La solution résultante est ensuite extraite successivement trois fois avec de petites quantités de NaHCO₃ 0,5 M, trois fois avec une solution de KHSO₄ à 50 g/l puis plusieurs fois avec H₂O à demi-saturée en NaCl. La phase organique est ensuite séchée sur sulfate de sodium anhydre. Après filtration (élimination de Na₂SO₄), on évapore le solvant et recristallise le résidu d'évaporation dans le mélange AcOEt/MeOMe (3/7) v/v. On obtient 3,5 g du produit attendu, sous forme de poudre de couleur blanche. F = 128-130°C.

Analyse (CCM sur gel de silice) :
Rf = 0,5 dans AcOEt/pyridine/AcOH/H₂O (20/4,5/3/1) v/v;
Rf = 0,69 dans CHCl₃/MeOH/AcOH (5/3/1) v/v.

### b) H-L-Arg-pNA, 2HBr

Dans un appareillage en verre et téflon, on charge 1 g (2,15 mmoles) de Z-L-Arg-pNA, HCl. On ajoute, sous atmosphère inerte (courant d'azote), successivement 8 ml de AcOH glacial, 2 ml d'anisol, 10 ml de HBr en solution (à 33 % p/v) dans AcOH. On laisse réagir pendant 1 h à RT sous atmosphère d'azote. Après ce laps de temps, le mélange réactionnel, qui est devenu homogène au cours de la déprotection, est précipité dans de l'éther (MeOMe ou EtOEt). Après décantation, le surnageant est écarté et le précipité est lavé plusieurs fois à l'éther. On recueille le précipité par filtration, le sèche sous vide sur KOH pendant 24 h. On obtient 0,95 g du produit attendu.

Analyse (CCM sur gel de silice) :
Rf = 0,04 dans AcOEt/pyridine/AcOH/H₂O (20/4,5/3/1,5) v/v
Rf = 0,38 dans BuOH/AcOH/H₂O (3/1/1) v/v.

### c) Boc-L-Pro-L-Arg-pNA, HBr

On dissout 1 g (2,19 mmoles) de H-L-Arg-pNA, 2HBr dans 10 ml de DMF puis on ajoute 0,854 ml (6,57 mmoles) de DIEA. Dans un autre récipient, on neutralise 4,71 mg (2,19 mmoles) de Boc-L-Pro-OH dissous dans 50 ml de DMF par 0,285 ml de DIEA. On mélange les deux solutions puis ajoute 970 mg de Bop tout en maintenant le mélange résultant à RT sous agitation, le pH étant maintenu entre 7,0 et 8,0 tout au long de la réaction par addition de faibles portions de DIEA. Au bout d'une heure, la réaction est terminée et le mélange réactionnel est évaporé à sec sous vide; le résidu d'évaporation est repris par un mélange AcOEt/MeOH et extrait par une solution aqueuse de NaHCO₃ 0,5 M. On sèche la phase organique sur sulfate de sodium, la concentre et précipite dans l'éther (MeOMe ou EtOEt). On obtient 877 mg (rendement : 70 %) du produit attendu.

Analyse (CCM sur gel de silice) :
Rf = 0,27 dans CHCl₃/MeOH/AcOH (20/3/1) v/v;
Rf = 0,71 dans CHCl₃/MeOH/AcOH (10/3/1) v/v.

### d) H-L-Pro-L-Arg-pNA, 2H-TFA

Dans un réacteur on introduit successivement 850 mg (1,485 mmoles) de Boc-L-Pro-L-Arg-pNA, HBr puis 6 ml de CH₂Cl₂ et 6 ml de H-TFA. Après 0,25 h de réaction, le mélange réactionnel est directement précipité dans l'éther. Après séchage du précipité résultant, on obtient 837 mg du produit attendu.

Analyse (CCM sur gel de silice) :
Rf = 0,56 dans BuOH/AcOH/H₂O (3/1/1) v/v;
Rf = 0,13 dans CHCl₃/MeOH/AcOH (5/3/1) v/v.

### e) L-THC-L-Pro-L-Arg-pNA, H-TFA

Selon le protocole opératoire décrit au point c) ci-dessus, à savoir réaction à RT pendant 1,5 h d'un mélange réactionnel comprenant (i) 800 mg (1,3 mmoles) de H-L-Pro-L-Arg-pNA, 2H-TFA, (ii) 190 mg (1,3 mmoles) de H-THC-OH, (iii) 1 ml de DIEA et (iv) 5,75 mg (1,3 mmoles) de Bop, dans le DMF sous agitation. Le mélange réactionnel est ensuite évaporé sous vide puis précipité dans EtOEt. Le précipité blanc obtenu est repris dans une quantité minimale d'un système solvant CHCl₃/MeOH/AcOH (5/3/1) v/v et chromatographié sur gel de silice au moyen du même système solvant. On récupère les fractions homogènes. Celles-ci sont rassemblées et évaporées. On obtient 410 g (rendement : 54 %) du produit attendu.

Analyse (CCM sur gel de silice) :
Rf = 0,23 dans CHCl₃/MeOH/AcOH (10/3/1) v/v;
Rf = 0,33 dans CHCl₃/MeOH/AcOH (5/3/1) v/v.

### PREPARATION II

### Obtention de L-THC-L-Pro-L-Arg-pNA, AcOH

### (Exemple 2)

A partir de L-THC-L-Pro-L-Arg-pNA, H-TFA obtenu selon la préparation I, on prépare le produit de l'exemple 2, le L-THC-L-Pro-L-Arg-pNA, AcOH par traitement sur résine échangeuse d'ions AMBERLITE^{R} IRA 401 S (préalablement acétylée), l'éluant étant un mélange MeOH/H₂O (3/2) v/v.

### PREPARATION III

On prépare le produit de l'exemple 1 en remplaçant la chromatographie sur gel de silice prévue au stade Ie par une HPLC (colonne HYPERSIL^{R} C 18 de granulométrie 3 micromètres). On obtient un produit plus pur que celui obtenu à la préparation I.

## Revendications

1. Composé peptidique caractérisé en ce qu'il est choisi parmi l'ensemble constitué par
(i) les tripeptides de formule
Y-A₁-A₂-A₃-R (I)
dans laquelle
Y représente H ou un groupe approprié bloquant l'extrémité N-terminale,
A₁ représente un reste aminoacide choisi parmi l'ensemble constitué par les restes (2-oxo-thiazolidin-4-yl)carbonyle [en abrégé : THC], (2-oxo-tétrahydro-1,3-thiazin-5-yl)carbonyle [en abrégé : TZC], thiazolidine-4-carbonyle [en abrégé : ATC] et (tétrahydro-1,3-thiazin-5-yl)carbonyle [en abrégé = AZC],
A₂ représente un reste aminoacide choisi parmi l'ensemble constitué par les restes Pro, 3Hyp et 4Hyp, où le groupe OH latéral de 3Hyp et 4Hyp est susceptible d'être protégé par un groupe protecteur éther ou ester,
A₃ représente un reste aminoacide choisi parmi l'ensemble constitué par les restes Arg et Lys, et R représente un moyen de marquage; et
(ii) leurs sels d'addition.

2. Composé peptidique selon la revendication 1, caractérisé en ce que le reste aminoacide A₁ est de configuration D ou L, et en ce que les restes aminoacides A₂ et A₃ sont de configuration L.

3. Composé peptidique selon la revendication 1, caractérisé en ce que Y est H.

4. Composé peptidique selon la revendication 1, caractérisé en ce que A₁ est L-THC.

5. Composé peptidique selon la revendication 1, caractérisé en ce que avec en ce qu'il est choisi parmi l'ensemble constitué par
(a) les composés tripeptidiques de formule
H-A₁-A₂-A₃-pNA (I')
dans laquelle
A₁ représente D-THC, D-TZC, D-ATC, D-AZC, L-THC, L-TZC, L-ATC ou L-AZC,
A₂ représente L-Pro, L-3Hyp ou L-4Hyp, et
A₃ représente L-Arg ou L-Lys; et
(b) leurs sels d'addition.

6. Composé peptidique selon la revendication 5, caractérisé en ce qu'il est choisi parmi l'ensemble constitué par le H-L-THC-L-Pro-L-Arg-pNA et ses sels d'addition.

7. Utilisation d'un composé peptidique selon la revendication 1 dans la détermination de la protéine C.

8. Procédé de détermination de la protéine C, caractérisé en ce que l'on met en contact dans un milieu biologique aqueux une quantité donnée d'un composé peptidique selon la revendication 1 avec un échantillon à tester contenant de la protéine C.

9. Procédé selon la revendication 8, caractérisé en ce que l'on met en contact dans un milieu biologique aqueux une quantité donnée d'un composé peptidique selon la revendication 1 avec un échantillon à tester contenant de la protéine C pendant au moins 0,25 h, à une température comprise entre 15 et 40°C.

10. Procédé de préparation d'un composé peptidique de formule I ou l'un de ses sels d'addition, ledit procédé, qui met en oeuvre des mécanismes de synthèse peptidique, étant caractérisé en ce qu'il comprend la réaction de 1 mole de dipeptide de formule
H-A₂-A₃-R (IV)
où A₂, A₃ et R sont définis comme indiqué ci-dessus dans la revendication 1, avec 1,0 à 1,3 moles d'aminoacide de formule
Y-A₃-T (V)
où Y et A₃ sont définis comme indiqué ci-dessus dans la revendication 1, et T représente OH, F, Cl ou Br.

11. Nécessaire pour la détermination de la Protéine C, caractérisé en ce qu'il comprend au moins un compose choisi parmi les tripeptides de formule I et leurs sels d'addition selon la revendication 1, et le cas échéant, un échantillon étalon de Protéine C et des milieux tamponnés de dilution.

## Claims

1. A peptide compound which is selected from the group consisting of
(i) the tripeptides of the formula
Y-A₁-A₂-A₃-R (I)
in which
Y is H or an appropriate group blocking the N-terminal end,
A₁ is an amino acid residue selected from the group consisting of (2-oxothiazolidin-4-yl)carbonyl [abbreviated to THC], (2-oxotetrahydro-1,3-thiazin-5-yl)carbonyl [abbreviated to TZC], thiazolidine-4-carbonyl [abbreviated to ATC] and (tetrahydro-1,3-thiazin-5-yl)carbonyl [abbreviated to AZC] residues,
A₂ is an amino acid residue selected from the group consisting of Pro, 3Hyp and 4Hyp residues, where the OH side-group of 3Hyp and 4Hyp is capable of being protected by an ether or ester protecting group,
A₃ is an amino acid residue selected from the group consisting of Arg and Lys residues, and
R is a labeling means; and
(ii) their addition salts.

2. A peptide compound according to claim 1 wherein the amino acid residue A₁ has the D or L configuration and wherein the amino acid residues A₂ and A₃ have the L configuration.

3. A peptide compound according to claim 1 wherein Y is H.

4. A peptide compound according to claim 1 wherein A₁ is L-THC.

5. A peptide compound according to claim 1 which is selected from the group consisting of
(a) the tripeptide compounds of the formula
H-A₁-A₂-A₃-pNA (I')
in which
A₁ is D-THC, D-TZC, D-ATC, D-AZC, L-THC, L-TZC, L-ATC or L-AZC,
A₂ is L-Pro, L-3Hyp or L-4Hyp and
A₃ is L-Arg or L-Lys; and
(b) their addition salts.

6. A peptide compound according to claim 5 which is selected from the group consisting of H-L-THC-L-Pro-L-Arg-pNA and its addition salts.

7. Use of a peptide compound according to claim 1 in the determination of Protein C.

8. A method of determining Protein C, wherein a given amount of a peptide compound according to claim 1 is brought into contact, in an aqueous biological medium, with a test sample containing Protein C.

9. A method according to claim 8 wherein a given amount of a peptide compound according to claim 1 is brought into contact, in an aqueous biological medium, with a test sample containing Protein C, for at least 0.25 h at a temperature of between 15 and 40°C.

10. A method of preparing a peptide compound of formula I or one of its addition salts, said method, which uses mechanisms of peptide synthesis, comprising reacting 1 mol of a dipeptide of the formula
H-A₂-A₃-R (IV)
in which A₂, A₃ and R are defined as indicated above in claim 1, with 1.0 to 1.3 mol of an amino acid of the formula
Y-A₃-T (V)
in which Y and A₃ are defined as indicated above in claim 1 and T is OH, F, Cl or Br.

11. A kit for the determination of Protein C, which comprises at least one compound selected from the tripeptides of formula I and their addition salts according to claim 1 and, if appropriate, a standard sample of Protein C and buffered dilution media.

## Patentansprüche

1. Peptidverbindung, dadurch gekennzeichnet, daß sie aus einer Gruppe ausgewählt ist, die von
(i) Tripeptiden der Formel
Y-A₁-A₂-A₃-R (I)
in der
Y H oder eine geeignete, das N-terminale Ende blockierende, Gruppe ist,
A₁ ein Aininosäurerest ist, der aus einer Gruppe ausgewählt ist, die von den Resten (2-Oxo-thiazolidin-4-yl) carbonyl [abgekürzt: THC],
(2-Oxo-tetrahydro-1,3-thiazin-5-yl) carbonyl [abgekürzt: TZC], Thiazolidin-4-carbonyl [abgekürzt: ATC] und (Tetrahydro-1,3-thiazin-5-yl)carbonyl [abgekürzt = AZC],
A₂ ein Aminosäurerest ist, der aus einer Gruppe ausgewählt ist, die von den Resten Pro, 3Hyp und 4Hyp gebildet ist, wobei die seitenständige Gruppe OH von 3Hyp und 4Hyp mittels einer Ether- oder Ester-Schutzgruppe schützbar ist und
A₃ einen Aminosäurerest bedeutet, der aus einer Gruppe ausgewählt ist, die von den Resten Arg und Lys gebildet ist, und R ein Markierungsmittel ist, und
(ii) Additionssalzen derselben
gebildet ist.

2. Peptidverbindung nach Anspruch 1, dadurch gekennzeichnet, daß der Aminosäurerest A₁ die Konfiguration D oder L aufweist und die Aminosäurereste A₂ und A₃ die Konfiguration L aufweisen.

3. Peptidverbindung nach Anspruch 1, dadurch gekennzeichnet, daß Y H ist.

4. Peptidverbindung nach Anspruch 1, dadurch gekennzeichnet, daß A₁ L-THC ist.

5. Peptidverbindung nach Anspruch 1, dadurch gekennzeichnet, daß sie aus einer Gruppe ausgewählt ist, die von
(a) Tripeptidverbindungen der Formel
H-A₁-A₂-A₃-pNA (I')
in der
A₁ D-THC, D-TZC, D-ATC, D-AZC, L-THC, L-TZC, L-ATC oder L-AZC,
A₂ L-Pro, L-3Hyp oder L-4Hyp und
A₃ L-Arg oder L-Lys bedeuten, und
(b) Additionssalzen derselben
gebildet ist.

6. Peptidverbindung nach Anspruch 5, dadurch gekennzeichnet, daß sie aus einer Gruppe ausgewählt ist, die von H-L-THC-L-Pro-L-Arg-pNA und Additionssalzen desselben gebildet ist.

7. Verwendung einer Peptidverbindung nach Anspruch 1 für die Bestimmung von Protein C.

8. Verfahren zur Bestimmung des Proteins C, dadurch gekennzeichnet, daß man in einem wäßrigen, biologischen Milieu eine definierte Menge einer Peptidverbindung nach Anspruch 1 mit einer zu untersuchenden, das Protein C enthaltenden Probe zusammenbringt.

9. Verfahren nach Anspruch 8, dadurch gekennzeichnet, daß man in einem wäßrigen, biologischen Milieu eine definierte Menge einer Peptidverbindung nach Anspruch 1 mit einer zu untersuchenden, das Protein C enthaltenden Probe während mindestens 0,25 h bei einer Temperatur zwischen 15 und 40°C zusammenbringt.

10. Verfahren zur Herstellung einer Peptidverbindung der Formel I oder eines Additionssalzes derselben, wobei in dem Verfahren die Abläufe der Peptidsynthese verwendet werden, dadurch gekennzeichnet, daß es die Reaktion von 1 mol Dipeptid der Formel
H-A₂-A₃-R (IV)
in der A₂, A₃ und R wie oben angegeben im Anspruch 1 definiert sind, mit 1,0 bis 1,3 mol einer Aminosäure der Formel
Y-A₃-T (V)
in der Y und A₃ wie oben im Anspruch 1 definiert sind, und T OH, F, Cl oder Br bedeuted, umfaßt.

11. Kit für die Bestimmung des Proteins C, dadurch gekennzeichnet, daß es mindestens eine Verbindung, die aus den Tripeptiden der Formel I und den Additionssalzen derselben gemäß Anspruch 1 ausgewählt ist, und gegebenenfalls eine Eichprobe des Proteins C sowie gepufferte Verdünnungsmittel umfaßt.
